(19) Europäisches Patentamt
European Patent Office
Office européen des brevets

(11) Numéro de publication: **0 163 582**
**B1**

(12) **FASCICULE DE BREVET EUROPEEN**

(45) Date de publication du fascicule du brevet: **26.09.90**

(51) Int. Cl.$^5$: **A 61 K 31/725**

(21) Numéro de dépôt: **85401072.5**

(22) Date de dépôt: **30.05.85**

(54) **Médicaments favorisant les propriétés d'écoulement du sang et leur utilisation en thérapeutique.**

(30) Priorité: **30.05.84 FR 8408570**

(43) Date de publication de la demande:
**04.12.85 Bulletin 85/49**

(45) Mention de la délivrance du brevet:
**26.09.90 Bulletin 90/39**

(84) Etats contractants désignés:
**BE CH DE GB IT LI LU NL SE**

(56) Documents cités:
**DE-A-2 747 378**
**FR-A-2 360 311**

**CHEMICAL ABSTRACTS, vol. 85, 1976, page 51, résumé no. 116872v, Columbus, Ohio, US; A. ERDI et al.: "Effect of low-dose subcutaneous heparin on whole-blood viscosity", & LANCET 1976, 2(7981),342-3**

(73) Titulaire: **CHOAY S.A.**
**48, Avenue Théophile-Gautier**
**F-75782 Paris Cédex 16 (FR)**

(72) Inventeur: **Lormeau, Jean-Claude**
**1, rue Joseph Delattre**
**F-76150-Maromme (FR)**
Inventeur: **Petitou, Maurice**
**27, rue Javelot**
**F-75013 Paris (FR)**
Inventeur: **Choay, Jean**
**21, rue St. Guillaume**
**F-75007 Paris (FR)**
Inventeur: **Toulemonde, Francis**
**4, rue de Noisy**
**F-78870 Bailly (FR)**

(74) Mandataire: **Peaucelle, Chantal et al**
**S.C. Ernest Gutmann - Yves Plasseraud 67, boulevard Haussmann**
**F-75008 Paris (FR)**

**EP 0 163 582 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

L'invention se rapporte à des médicaments favorisant les propriétés d'écoulement du sang et à leur utilisation en thérapeutique.

La demanderesse a déjà décrit des médicaments renfermant des fractions mucopolysaccharidiques dotées de propriétés biologiques leur permettant de jouer un rôle régulateur vis-à-vis de la coagulation sanguine, plus particulièrement dans le sens d'un retard à la coagulation. Cette action régulatrice s'exerce par la mise en jeu d'actions inhibitrice plus sélectives que celles de l'héparine, à l'égard d'un nombre plus réduit de facteurs de la coagulation, plus particulièrement du facteur X activé (facteur Xa).

Ces mucopolysaccharides peuvent être obtenus par fractionnement alcoolique à partir de l'héparine ou de fractions comportant des constituants hépariniques tels que récupérés par extraction à partir de tissus ou d'organes d'animaux, en particulier de mammifères (ou notera que le terme "héparine" est utilisé pour désigner indifféremment une héparine commerciale de qualité pharmaceutique ou une héparine obtenue par extraction).

Ces fractions sont caractérisées notamment par les propriétés suivantes:

elles possèdent un poids moléculaire d'environ 2000 à 10 000 daltons;

elles sont solubles dans un milieu hydroalcoolique (eau-ethanol) ayant un titre de 55—61°GL;

elles tendent à l'insolubilité dans un milieu eau-alcool ayant une teneur en alcool plus élevée;

elles sont insolubles dans l'alcool pur;

elles possèdent un rapport du titre anti-Xa (mesuré par le titre Yin-Wessler) au titre USP, d'au moins 2, notamment d'au moins 3, et même supérieur à 6.

Les inventeurs ont à présent constaté que ces fractions exercent une action puissante sur le comportement rhéologique du sang.

Les travaux développés sur ces résultats ont permis de mettre en évidence une telle action, d'une manière générale, chez d'autres fractions et compositions mucopolysaccharidiques répondant aux caractéristiques générales ci-dessus.

Dans la suite du texte, ces produits seront également désignés par le terme oligosaccharide.

L'invention vise donc l'utilisation d'oligosaccharides pour l'obtention d'un médicament permettant de favoriser, de manière importante, l'écoulement du sang. Elle vise également leur utilisation sous différentes formes d'administration pour combattre les facteurs responsables de l'hyperviscosité sanguine.

Conformément à l'invention, ces oligosaccharides actifs sur la viscosité sanguine, sont caractérisés en ce qu'ils correspondent ou comportent des fragments d'héparine d'un poids moléculaire inférieur à 10 000, possédant un rapport du titre Yin-Wessler au titre USP d'au moins 2 et présentant les propriétés de solubilité rappelées ci-dessus, ou de leurs sels pharmacologiquement acceptables.

Dans une famille préférée, ces médicaments renferment au moins une fraction mucopolysaccharidique selon le brevet FR—A—2 440 376 du 6 novembre 1978 au nom de la demanderesse.

Des médicaments particulièrement efficaces pour l'activité considérée comprennent des fractions mucopolysaccharidiques ayant un poids moléculaire d'environ 2000 à 8000, notamment un poids moléculaire moyen de l'ordre de 3500 à 5000.

Les spectres RMN ($^{13}C$: radiation de 20 MHz) de solutions de fractions préférées, dissoutes dans de l'eau deutériée, montrent des signaux ayant les caractéristiques suivantes:

23.4 ppm: $CH_3$ (groupe NH-acétyle)

54,7 ppm: carbone en position 2 des motifs glucosamineacétyle

59,2 et 58,8 ppm: carbone en position 2 des motifs sulfate glucosamine (le pic dans la région des 58 ppm n'apparaît pas dans des conditions de mesure identiques avec l'héparine)

61,7 ppm: carbone en position 6 avec des groupes —OH

67,4 ppm: carbone en position 6 avec des groupes —O-sulfate

98,3** ppm: carbone en position 1

100,00 ppm**: carbone en position 1 des motifs acide iduronique sulfaté

≃ 103 ppm: carbone en position 1 des motifs acide glucuronique

Des fractions mucopolysaccharidiques préférées possèdent un rapport des titres γω/USP d'environ 3 à 5.

L'invention vise particulièrement l'utilisation, pour favoriser les propriétés d'écoulement du sang, d'une fraction du type de celle de l'exemple 1 du brevet FR—A—2 440 376 dont question ci-dessus.

Une telle fraction appelée ci-après CY 216 possède un rapport du titre Yin-Wessler au titre USP de l'ordre de 3,55. Elle est obtenue par extraction alcoolique à partir d'héparine.

D'autres oligosaccharides de cette famille comprennent les fractions obtenues par fractionnements alcooliques successifs suivis, le cas échéant, d'opérations de fractionnements selon le poids moléculaire telles que des gel-filtrations ou des gel-perméations.

Les produits obtenus sont caractérisés par des rapports des titres Yin-Wessler-USP supérieurs à 10, notamment de l'ordre de 13 à 16 avec des titres Yin-Wessler supérieurs à 130 u/mg, notamment de l'ordre de 135 à 160 u/mg.

Par purification de ces fractions à l'aide d'antithrombine III ou AT III, selon le premier certificat d'addition FR—A—2 461 719 du 20.7.1979 au brevet principal FR—A—2 440 376, on obtient des

compositions de mucopolysaccharides dont le titre Yin-Wessler est au moins égal à 300 u/mg, avantageusement supérieur à 1300 u/mg avec des rapports du titre Yin-Wessler au titre USP d'au moins 6 et pouvant dépasser 65 environ.

Dans une autre famille préférée, les médicaments utilisés conformément à l'invention renferment des oligosaccharides du type des compositions mucopolysaccharidiques décrites dans la demande de brevet EP—A—37319 du 20 mars 1980.

Il s'agit de compositions formées d'un mélange de chaînes possédant plus spécialement un poids moléculaire moyen de 3000 à 6000 daltons environ.

Ces compositions sont avantageusement obtenues par dépolymérisation ménagée d'héparine à l'aide d'un agent chimique. L'utilisation d'acide nitreux, dans les conditions rapportées dans cette deuxième demande de certificat d'addition conduit à des chaînes oligosaccharidiques avec des motifs terminaux de structure 2,5-anhydromannose. Par traitement approprié, ces structures terminales sont avantageusement transformées en groupes plus stables de structure 2,5-anhydro-mannitol ou acide 2,5-anhydromannonique.

Des médicaments préférés de cette famille renferment des compositions du type de celles de l'exemple 2 de cette demande de brevet EP qui se terminent par des motifs de structure 2,5-anhydro mannitol et possèdent un poids moléculaire de l'ordre de 2000—2500 daltons. Leur titre YW peut être supérieur à 200 u/mg. Le rapport YW/USP est supérieur à 2, et peut dépasser 10 selon les variantes de réalisation.

Des compositions de ce type, telles qu'obtenues par dépolymérisation autorégulée de l'héparine selon le procédé décrit dans la demande de brevet FR—A—2 503 714 du 10/04/1982, sont également avantageusement utilisées pour la préparation des oligosaccharides utilisés selon l'invention. Ces compositions possèdent au moins 98% de chaînes avec des motifs terminaux de structure 2,5 anhydromannose et présentent une grante homogénéité.

D'autres oligosaccharides de faible poids moléculaire par rapport à l'héparine possèdent également des propriétés avantageuses sur la viscosité sanguine.

Il en est ainsi des oligosaccharides du type de ceux décrits dans la demande EP—A—27089 du 6 octobre 1980.

Ces oligosaccharides sont caractérisés notamment par le fait qu'ils ne renferment pas plus de 8 motifs. saccharidiques, qu'ils possèdent une affinité spécifique pour l'ATIII et un rapport du titre Yin-Wessler au titre USP d'au moins 30.

L'activité USP observée est pratiquement nulle alors que le titre Yin-Wessler peut dépasser de dix fois celui de l'héparine et même atteindre une valeur de 2000 u/mg environ avec un octasaccharide appelé ABCDEFGH obtenu par dépolymérisation enzymatique de l'héparine de structure:

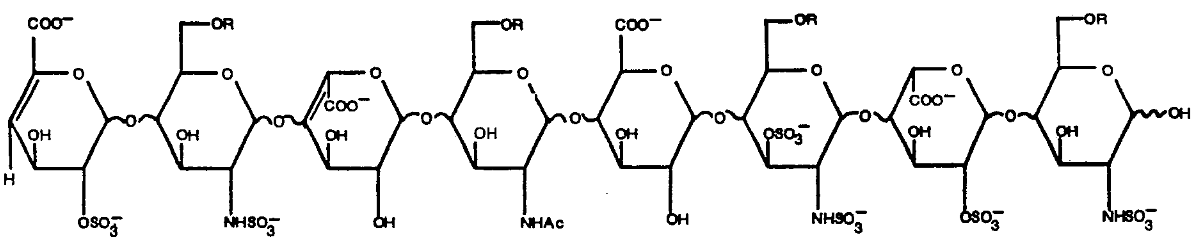

A B D E F G H

Des compositions hexasaccharidiques de grande homogéniété, du type de celles décrites dans le brevet EP—A 064 452 du 26/04/1982 présentent également un grand intérêt pour l'élaboration des compositions utilisées selon l'invention.

Ces compositions à base de chaînes de structure C'DEFGH obtenues selon un procédé ménagé de dépolymérisation enzymatique possèdent notamment des titres Yin-Wessler supérieurs à 2000 u/mg et des titres Yin-Wessler supérieurs à 2000 u/mg et des titres USP ou APTT de l'ordre de 10 ou inférieurs.

Dans une autre famille préférée de médicaments de l'invention, les oligosaccharides de faible poids moléculaires sont constitués par les produits décrits dans la demande EP—A 82793 du 17/01/1983. Ces produits obtenus par voie de synthèse possèdent un degré de pureté élevé et peuvent comporter étant donné leur mode d'obtention les substituants souhaités, favorisant une action donnée. D'une manière générale, ces oligosaccharides possèdent la structure de fragments d'héparine ou d'héparan-sulfate ou sont constitués par de tels fragments à motifs alternés sucre aminé-acide uronique (ou analogue de structure) ou l'inverse.

Parmi les produits décrits, on citera le pentasaccharide de structure DEFGH de formule:

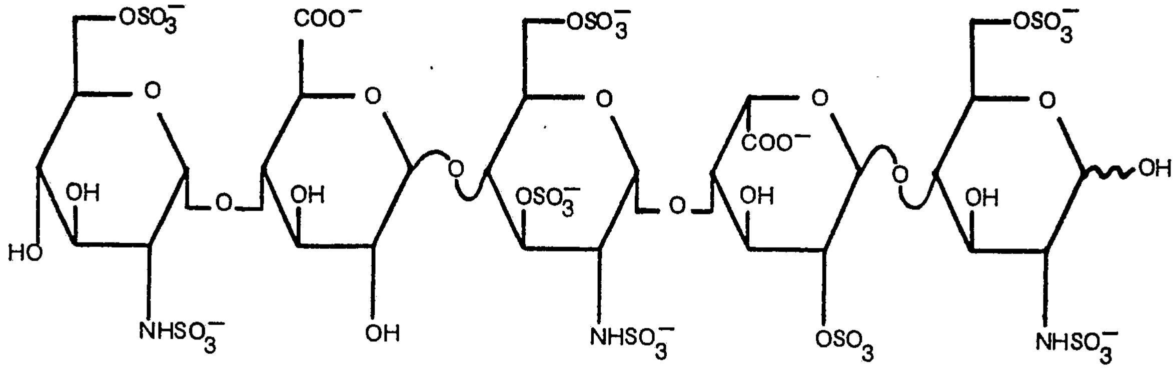

D'autres oligosaccharides qui se sont révélés précieux pour l'élaboration des médicaments utilisés selon l'invention correspondent à ceux décrits dans la demande FR 2 564 468 publiée le 25.11.85.

Selon leur définition la plus générale, ces oligosaccharides comprennent 4 à 12 motifs choisis parmi les motifs sucre aminé-acide uronique, ou l'inverse, et contiennent un enchaînement tétrasaccharidique de structure DEFG correspondant à la formule

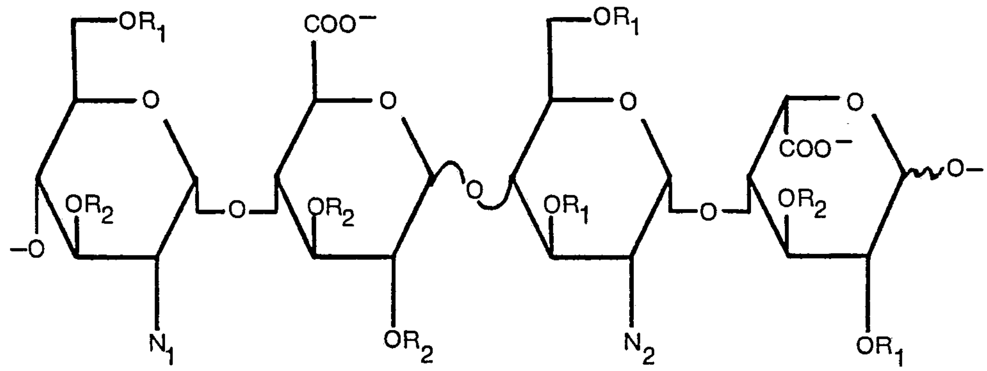

dans laquelle

les radicaux $R_1$, identiques ou différents les uns des autres, représentent un anion minéral, en particulier un groupe sulfate ou un groupe phosphate,

$R_2$ présente l'une des significations données pour $R_1$ ou représente un atome d'hydrogène,

$N_1$ et $N_2$, identiques ou différents l'un de l'autre représentent un groupe fonctionnel amine, en particulier, sous forme de sel avec un anion minéral tel que défini ci-dessus, ou substitué par un groupe acyle —$COR_3$ où $R_3$ représente un radical alcoyle et leurs sels.

Un oligosaccharide préféré de cette famille présente la structure DEFG et répond à la formule suivante:

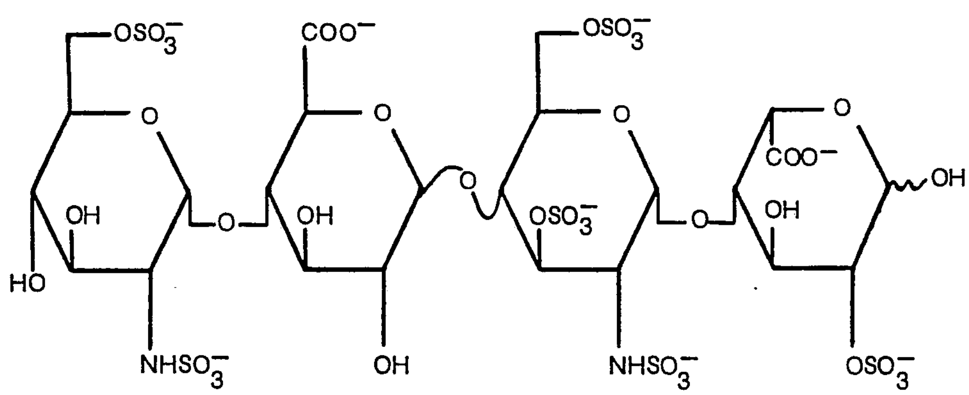

Selon une variante de l'invention, les oligosaccharides mis en oeuvre dans les différentes familles de médicaments évoquées ci-dessus sont utilisés sous des formes de conjugués avec l'AT—III unis par liaison covalente ou comme décrit dans la demande de brevet FR—A—2 548 189 du 2/07/1984, sous forme de complexes résultant de l'affinité de ces oligosaccharides pour l'AT—III.

Comme déjà souligné, ces oligosaccharides de faible poids moléculaire exercent une action de grand intérêt sur le comportement rhéologique du sang diminuant considérablement la viscosité sanguine.

Ces propriétés permettent leur utilisation dans divers syndromes d'hyperviscosités où jusqu'alors il était fait essentiellement appel à des techniques d'hémodilution d'effets de courte durée.

On citera les syndromes d'hyperviscosités associées à un certain nombre de maladies en particulier des artériopathies, des insuffisances cérabrales chroniques chez les sujets âgés, les problèmes d'hyperviscosités rencontrés chez lesbrûlés, les syndromes d'hyperviscosités associées à des états d'insuffisances artérielles sub-aigües, par exemple, dans des cas d'ischémie cérébrale (thromboses localisées dans le cerveau) d'insuffisance cardiaque (état de mal angineux).

L'étude de l'action de ces médicaments a montré, sur un tout autre plan, qu'ils permettent d'éviter les risques de déminéralisation rencontrés sur un traitement à long terme, par exemple dans le cas de femmes enceintes ou de malades thrombo-emboliques réagissant mal aux anticoagulants oraux et soumis de ce fait à un traitement à long terme avec de l'héparine.

Les oligosaccharides ci-dessus sont associés aux excipients pharmaceutiques habituels. La bonne absorption de ces oligosaccharides par la muqueuse du transit intestinal permet leur utilisation sous de nombreuses formes galéniques.

L'invention concerne, en particulier, les médicaments dans lesquels le véhicule est approprié pour l'administration par voie orale. Des formes d'administration correspondantes comprennent, des comprimés, capsules, dragées, granulés, pilules et autres. L'administration sous forme de liposomes des différents oligosaccharides de bas poids moléculaires évoqués ci-dessus s'avère particulièrement appropriée pour le traitement des syndromes d'hyperviscosité.

D'autres compositions pharmaceutiques comprennent ces oligosaccharides en association avec les excipients appropriés pour l'administration par voie rectale. Des formes d'administration correspondantes sont constituées par des suppositoires.

En particulier, l'invention concerne également des compositions pharmaceutiques injectables, stériles ou stérilisables, pour l'administration par voie intraveineuse, intramusculaire ou sous-cutanée.

D'une manière générale, l'administration d'environ 3 à $10 \times 10^3$ u anti-Xa, (Yin-Wessler), notamment de l'ordre de $5 \times 10^3$ u anti-Xa environ deux ou trois fois par jour apparaît satisfaisante.

Ces posologies seront cependant adaptées en fonction de la condition du patient. Etant donné la parfaite inocuité de ces produits, des doses plus importantes peuvent être administrées sans effet néfaste.

Afin d'illustrer l'invention, on rapporte dans l'exemple qui suit les résultats obtenus sur des volontaires sains auxquels on a administré des médicaments renfermant des oligosaccharides tels que définis ci-dessus.

La figure unique comporte les courbes de la variation de la viscosité du sang total de sujets en fonction du temps après administration de sérum physiologique, d'héparine et d'un médicament de l'invention.

## Exemple

Etude de l'effet d'un médicament renfermant du CY 216 sur la viscosité du sang de sujets en bonne santé

Les résultats qui suivent concernant cette étude ont porté sur 6 sujets en bonne santé auxquels on a administré, par voie intraveineuse, après une nuit de jeune, à une semaine d'intervalle, 70 unités anti-Xa/kg de CY 216, d'héparine, puis d'un volume équivalent de sérum physiologique.

Préalablement à l'administration des médicaments, puis 30, 120 et 240 mn après cette administration, on a prélevé des échantillons sanguins et déterminé la viscosité à différentes vitesses de cisaillement à l'aide d'un viscosimètre rotatif classique.

Les résultats obtenus sont rapportés sur la figure 1 dans laquelle les courbes a) à c) correspondent respectivement aux mesures effectuées dans le cas de l'administration du CY 216, d'héparine et de sérum physiologique.

L'examen de cette figure montre une diminution importante de la viscosité du sang total lors de l'administration du CY 216, mettant en évidence l'efficacité de ce produit sur les propriétés d'écoulement du sang.

De fortes diminutions de la viscosité du sang ont été également observées en utilisant à la place du CY 216 des oligosaccharides de faible poids moléculaire obtenus par dépolymérisation nitreuse ou enzymatique tels que le produit désigné ci-dessus par CY 222 ou encore des octasaccharides du type ABCDEFGH et des hexasaccharides du type CDEFGH.

De même, les produits obtenus par voie de synthèse tels que le pentasaccharide du type DEFGH, le tétrasaccharide de type DEFG ou d'autres oligosaccharides contenant au moins une séquence disaccharidique de l'octasaccharide A—H ci-dessus se sont révélés d'une grande efficacité.

D'autres essais ont été réalisés sur des patients souffrant de maladies vasculaires. L'administration des oligosaccharides à raison de 100 unités anti-Xa/kg trois fois parjour durant un mois a permis d'agir de manière favorable sur l'état de ces patients.

Exemples de Formes D'Administration

Comprimés enrobés

Composition par unité de prise:

| | |
|---|---|
| Produit actif | 0,200 g |
| Amidon de maïs STARX® 1500 | 0,140 g |
| Elcéma® 150 | 0,210 g |
| Béhénate de glycérol (Compritol® 888) | 0,0175 g |
| Stéarate de calcium | 0,007 g |
| Aérosil 972® | 0,0035 g |
| Encompress spécial q.s.p. | 0,700 g |
| Enrobage film gastro-résistant avec: | |
| Eudragit® L 100—55 dans: Isopropanol 40 p.100<br>Acétone, 60 p.100 | |
| Liposomes | |
| phosphatidycholine | 26 mg |
| cholestérol | 13 mg |
| NaCl à 9%o produit actif | 10 mg |

**Revendications**

1. Utilisation, pour l'obtention d'un médicament destiné au traitement de syndromes d'hyperviscosité, l'oligosaccharides correspondant à ou comportant des fragments d'héparine d'un poids moléculaire inférieur à 10 000 daltons, possédant un rapport du titre YinWessler au titre USP d'au moins 2, solubles dans un milieu hydro-alcoolique (eau/ethanol) ayant un titre de 55—61°GL, tendant à l'insolubilité dans un milieu eau-alcool ayant une teneur en alcool plus élevée, insolubles dans l'alcool pur.

2. Utilisation selon la revendication 1, caractérisée en ce que les oligosaccharides correspondent à

a) une fraction mucopolysaccharidique, du type de celles isolées par extraction à partir de l'héparine ayant un poids moléculaire d'environ 2 000 à 8 000 daltons, notamment un poids moléculaire moyen de l'ordre de 3 500 à 5 000 daltons et un rapport des titres YW/USP de l'ordre de 3 à 5, ou encore une fraction de ce type résultant de plusieurs fractionnements alcooliques suivis, le cas échéant, d'opérations de fractionnement selon le poids moléculaire telles qu'une filtration sur gel ou une perméation sur gel, ces fractions ayant des rapports YW/USP de l'ordre 13 à 16, avec des titres YW supérieurs à 130 u/mg, en particulier de l'ordre de 135 à 160 u/mg;

b) une fraction du type de celles obtenues par purification des fractions précédentes sur ATIII, ayant un titre YW au moins égal à 300 u/mg, avantageusement supérieur à 1 300 u/mg avec des rapports des titres YW/USP d'au moins 6, pouvant dépasser 65 environ,

c) une composition mucopolysaccharidique formée d'un mélange de chaînes possédant un poids moléculaire moyen d'environ 3000 à 6000 daltons et comportant des motifs terminaux de structure 2,5-anhydromanno, de préférence des groupes 2,5-anhydromannitol ou acide 2,5-anhydromannonique;

d) une composition mucopolysaccharidique du type de celles obtenues par dépolymérisation autorégulée de l'héparine, formée avantageusement d'un mélange de chaînes dont au moins 98% possèdent une structure 2,5-anhydromanno,

e) un oligosaccharide ne renfermant pas plus de 8 motifs saccharidiques, possédant une affinité spécifique pour l'ATIII et un rapport des titres YW/USP d'au moins 30, plus spécialement un octasaccharide de structure ABCDEFGH

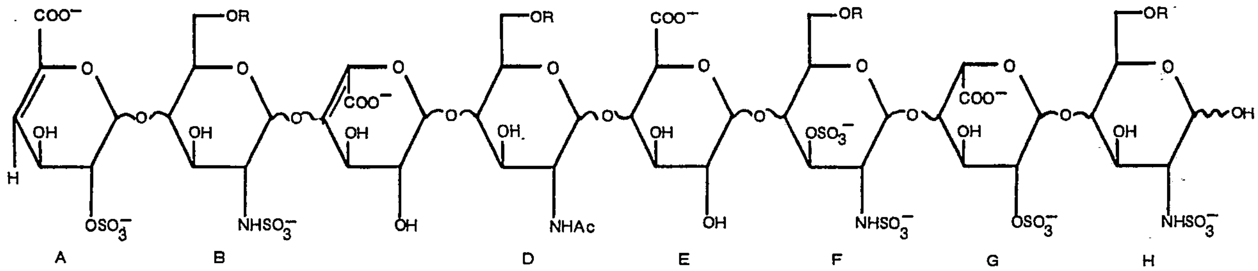

le titre USP de cet octasaccharide étant pratiquement nul et son titre YW supérieur de 10 fois à celui de l'héparine, pouvant même atteindre des valeurs de l'ordre de 2000 u/mg,

f) une composition hexasaccharidique de grande homogénéité à base de chaînes de structure C'DEFGH

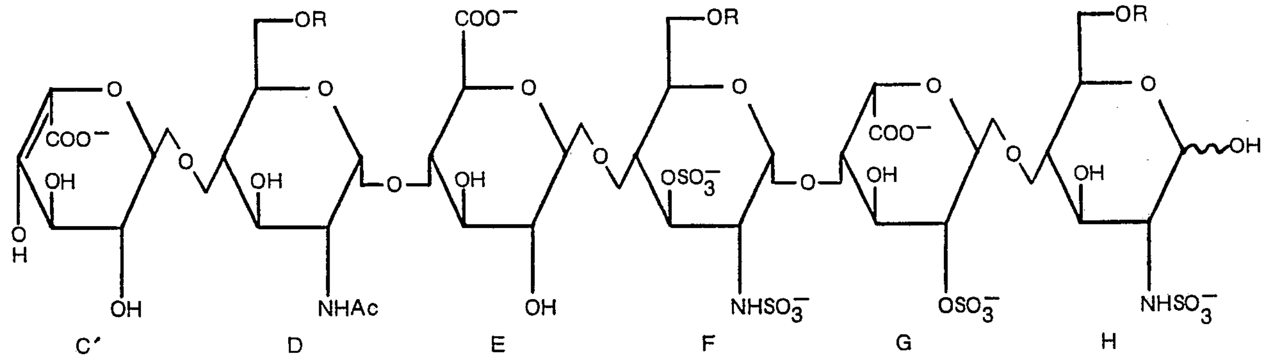

cette composition ayant un titre YW supérieur à 2000 u/mg et un titre USP ou APTT de l'ordre de 10 ou inférieur,

g) un oligosaccharide de faible poids moléculaire tels que ceux obtenus par voie de synthèse, en particulier le pentasaccharide de structure DEFGH de formule

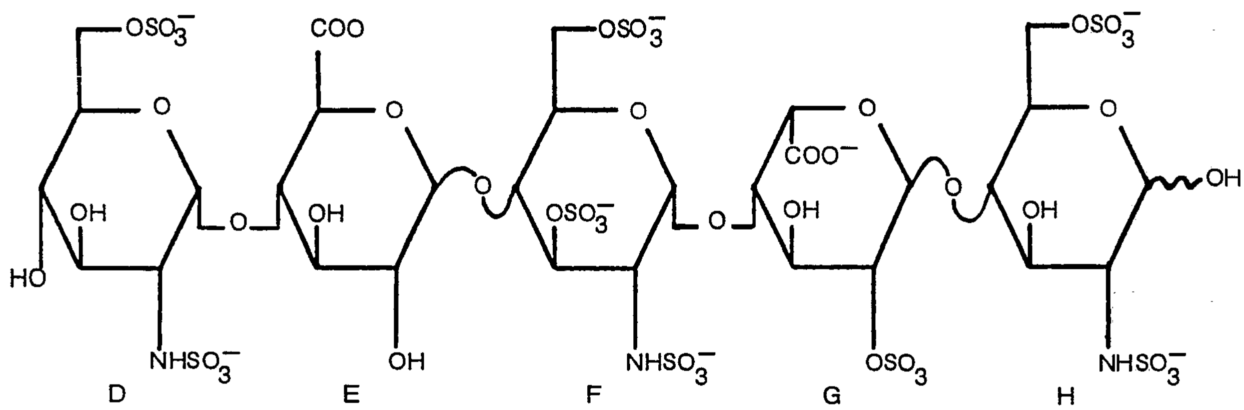

h) un oligosaccharide comprenant 4 à 12 motifs choisis parmi les motifs sucre aminé-acide uronique, ou l'inverse, et contenant un enchaînement tétrasaccharidique de structure DEFG

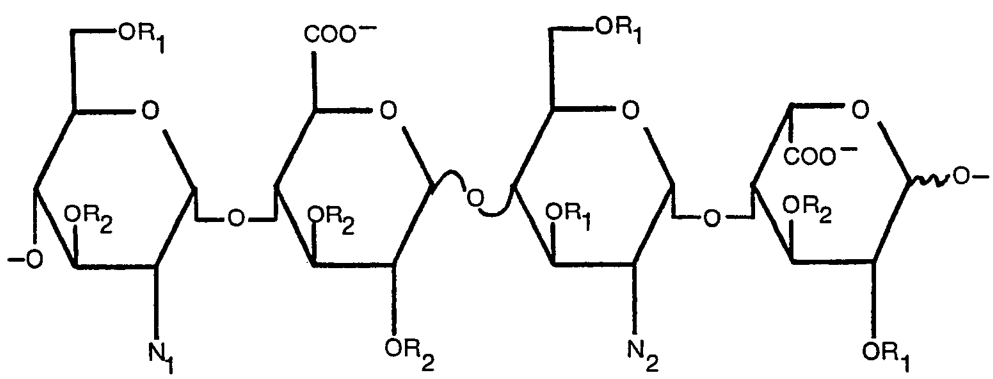

dans laquelle

les radicaux $R_1$, identiques ou différents les uns des autres, représentent un anion minéral, en particulier un groupe sulfate ou un groupe phosphate,

$R_2$ présente l'une des significations données pour $R_1$ ou représente un atome d'hydrogène,

$N_1$ et $N_2$, identiques ou différents l'un de l'autre représentent un groupe fonctionnel amine, en particulier sous forme de sel avec un anion minéral tel que défini ci-dessus, ou substitué par un groupe aryle —$COR_3$ où $R_3$ représente un radical alcoyle et leurs sels

i) un oligosaccharide ou une fraction tel que défini ci-dessus sous forme de conjugué avec l'ATIII ou sous forme de complexes d'affinité.

3. Utilisation selon la revendication 2, caractérisée en ce que les oligosaccharides correspondent à une fraction de mucopolysaccharide selon la revendication 2a présentant en solution deutériée, en RMN (13C, radiation de 20 MHz), les signaux suivants:

23,4 ppm: $CH_3$ (groupe NH-acétyle)

54,7 ppm: carbone en position 2 des motifs glucosamine-acétyle,

59,2 et 58,8 ppm: carbone en position 2 des motifs sulfate glucosamine (le pic dans la région des 58 ppm n'apparaît pas dans des conditions de mesure identiques avec l'héparine)

61,7 ppm: carbone en position 6 avec des groupes —OH,

67,4 ppm: carbone en position 6 avec des groupes —O-sulfate,

98,3** ppm: carbone en position 1

100,00 ppm**: carbone en position 1 des motifs acide iduronique sulfaté

≃ 103 ppm: carbone en position 1 des motifs acide glucuronique.

4. Utilisation selon l'une quelconque des revendications 1 à 3, caractérisée en ce que l'oligosaccharide est une fraction mucopolysaccharidique ayant un poids moléculaire moyen de l'ordre de 4.500 et un rapport de titres YW/USP voisin de 3,55.

5. Utilisation selon l'une quelconque des revendications précédentes, caractérisée en ce que l'oligosaccharide est mis en oeuvre en association avec un véhicule approprié pour l'administration orale.

**Patentansprüche**

1. Verwendung von Oligosacchariden zur Herstellung eines Medikaments, das zur Behandlung von Hyperviskositätssyndromen bestimmt ist, die Fragmenten von Heparin entsprechen oder sie enthalten, mit einem Molekulargewicht unter 10 000 Dalton, die ein Verhältnis von YinWessler-Titer zu USP-Titer von mindestens 2 besitzen, in einem Wasser-Alkohol-Milieu (Wasser/Ethanol) mit einem Titer von 55—61°GL löslich sind, in einem Wasser-Alkohol-Milieu, das einen höheren Alkoholgehalt hat zur Unlöslichkeit tendieren, in reinem Alkohol unlöslich sind.

2. Verwendung gemäß Anspruch 1, dadurch gekennzeichnet, daß die Oligosaccharide entsprechen

a) einer Mucopolysaccharidfraktion wie sie durch Extraktion ausgehend von Heparin isoliert werden, die ein Molekulargewicht von ungefähr 2 000 bis 8 000 Dalton hat, insbesondere ein mittleres Molekulargewicht in der Größenordnung von 3 500 bis 5 000 Dalton und ein Verhältnis der Titer YW/USP in der Größenordnung von 3 bis 5 oder ferner eine Fraktion von der Art, die aus mehreren aufeinanderfolgenden alkoholischen Fraktionierungen resultieren, gegebenenfalls von Fraktionierungen nach dem Molekulargewicht wie einer Gel-Filtration oder Gel-Permeation, wobei diese Fraktionen Verhältnisse YW/USP in der Größenordnung 13 bis 16, mit YW-Titern über 130 µ/mg, insbesondere in der Größenordnung von 135 bis 160 µ/mg haben;

b) einer Fraktion, wie sie durch Reinigung der vorhergenannten Fraktionen über ATIII erhalten werden, mit einem YW-Titer mindestens gleich 300 µ/mg, bevorzugt über 1 300 µ/mg mit Verhältnissen der Titer YW/USP von mindestens 6, die 65 ungefähr überschreiten können,

c) einer Mucopolysaccharidkomposition gebildet aus einer Mischung von Ketten, die ein mittleres Molekulargewicht von ungefähr 3 000 bis 6 000 Dalton besitzen und Endgruppen mit der 2,5-Anhydromanno-Struktur tragen, bevorzugt 2,5-Anhydromannitol-Gruppen oder 2,5-Anhydromannonsäure-Gruppen;

d) einer Mucopolysaccharidkomposition wie sie durch selbstregulierte Depolymerisation von Heparin erhalten werden, vorteilhafterweise gebildet aus einer Mischung von Ketten, von denen mindestens 98% eine 2,5-Anhydromanno-Struktur besitzen,

e) einem Oligosaccharid, das nicht mehr als 8 Saccharideinheiten enthält, eine spezifische Affinität für ATIII besitzt und ein Verhältnis der Titer YW/USP von mindestens 30, insbesondere ein Octasaccharid der Struktur ABCDEFGH

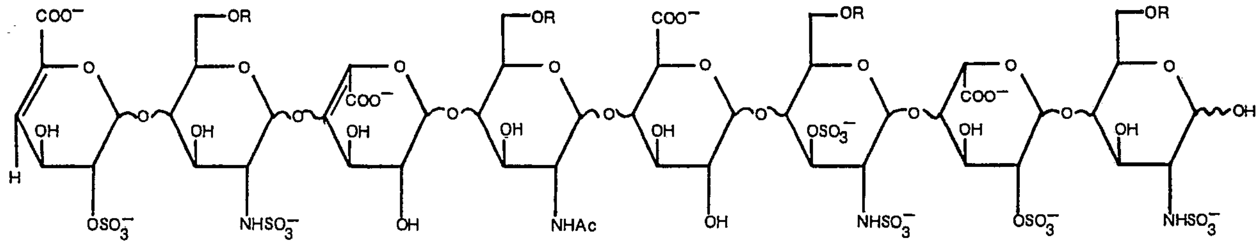

A B D E F G H

wobei der USP-Titer dieses Octasaccharids praktisch Null ist und sein YW-Titer 10fach größer als der des Heparins ist und sogar Werte der Größenordnung von 2 000 µ/mg erreichen kann,

f) einer Hexasaccharidkomposition von großer Homogenität ausgehend von Ketten der Struktur C'DEFGH

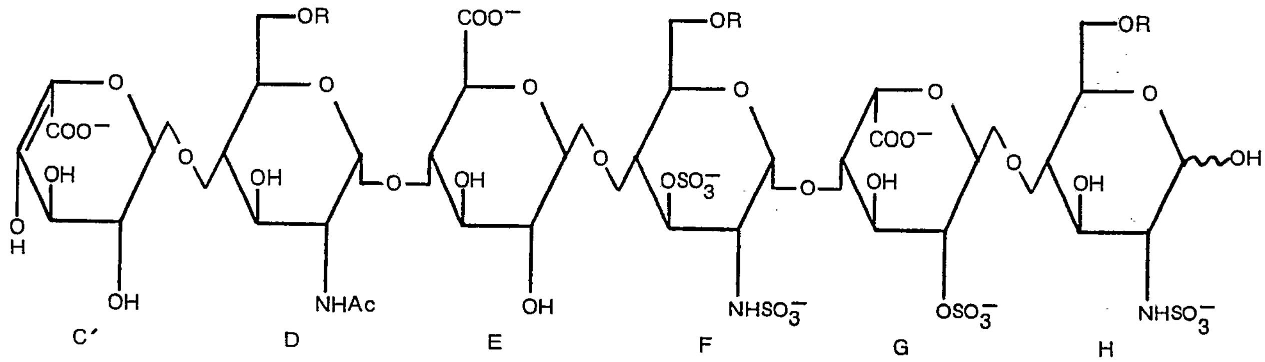

C' D E F G H

wobei diese Komposition einen YW-Titer über 2 000 µ/mg und einen USP-Titer oder APTT-Titer in der Größenordnung von 10 oder weniger hat,

g) einem Oligosaccharid von niedrigem Molekulargewicht wie sie durch Synthese erhalten werden, insbesondere das Pentasaccharid der Struktur DEFGH der Formel

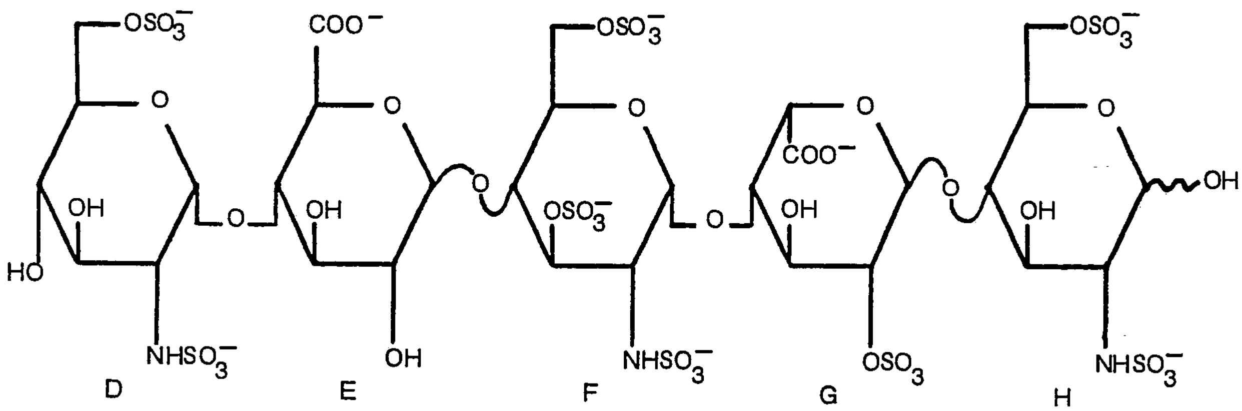

D E F G H

h) einem Oligosaccharid, das 4 bis 12 Einheiten umfaßt, die aus den Einheiten Aminozucker-Uronsäure, oder umgekehrt, ausgewählt sind, und eine Tetrasaccharid-Verkettung der Struktur DEFG enthalten

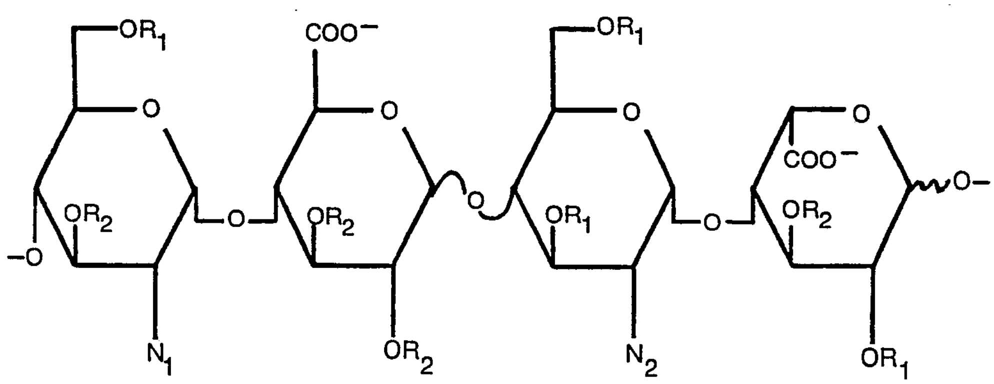

in der

die Radikale $R_1$, die identisch oder voneinander verschieden sind, ein Mineralanion darstellen, insbesondere eine Sulfatgruppe oder eine Phosphatgruppe,

$R_2$ eine der angegebenen Bedeutungen für $R_1$ hat oder ein Wasserstoffatom darstellt,

$N_1$ und $N_2$, identisch oder voneinander verschieden, eine funktionelle Amingruppe darstellen, insbesondere als Salz mit einem Mineralanion wie es oben definiert ist oder substituiert durch eine Acylgruppe —$COR_3$ wo $R_3$ ein Alkylradikal und ihre Salze darstellt,

i) einem Oligosaccharid oder einer Fraktion wie sie oben definiert wurde konjugierter Form mit ATIII oder als Affinitätskomplexe.

3. Verwendung gemäß Anspruch 2, dadurch gekennzeichnet, daß die Oligosaccharide entsprechen: einer Fraktion von Mucopolysacchariden gemäß Anspruch 2a, das in deuterierter Lösung im NMR ($^{13}C$) zeigt

23,4 ppm: $CH_3$ (NH-Acetylgruppe)

54,7 ppm: Kohlenstoff in Position 2 der Glucosaminacetyleinheiten

59,2 und 58,8 ppm: Kohlenstoff in Position 2 der Glucosaminsulfateinheiten (der Peak im Bereich 58 ppm erscheint nicht unter identischen Meßbedingungen mit Heparin)

61,7 ppm: Kohlenstoff in Position 6 mit —OH-Gruppen,

67,4 ppm: Kohlenstoff in Position 6 mit —O-Sulfat-Gruppen,

98,3** ppm: Kohlenstoff in Position 1,

100,00 ppm**: Kohlenstoff in Position 1 der sulfatierten Iduronsäureeinheiten,

103 ppm: Kohlenstoff in Position 1 der Glucuronsäureeinheiten.

4. Verwendung gemäß einem der Ansprüche 1 bis 3, dadurch gekennzeichnet, daß das Oligosaccharid eine Mucopolysaccharidfraktion ist, die ein mittleres Molekulargewicht in der Größenordnung von 4 500 hat und ein Verhältnis der Titer YW/USP nahe von 3,55.

5. Verwendung gemäß einem der vorhergehenden Ansprüche, dadurch gekennzeichnet, daß das Oligosaccharid angewendet wird in Verbindung mit einem geeigneten Transportmittel für die orale Verabreichung.

**Claims**

1. Use for obtaining a drug intended for the treatment of hyperviscosity syndroms, of oligosaccharides corresponding to or comprising heparin fragments having a molecular weight lower than 10,000 daltons, having a Yin-Wessler/USP ratio of at least 2, soluble in a water-alcoholic medium (water/ethanol) having a titer of 55°—61° GL, tending to insolubility in a water alcohol medium, having a higher alcohol content, insoluble in pure alcohol.

2. Use according to claim 1, wherein said oligosaccharides correspond to:

a) a mucopolysaccharidic fraction, of the type of those isolated by extraction from heparin having a molecular weight of about 2000 to 8000 daltons, particularly an average molecular weight of about 3500 to 5000 daltons and a ratio of the YW/USP titers of about 3 to 5, or again a fraction of said type resulting from several alcoholic fractionations optionally followed by fractionation operations according to the molecular weight such as a gel filtration or a gel permeation, said fractions having YW/USP ratios of the order of 13 to 16 with YW titers of at least 130 u/mg, particularly of the order of 135 to 160 u/mg;

b) a fraction of the type of those obtained by purification on AT III of the previous fractions, having a YW titer of at least 300 u/mg, advantageously higher than 1300 u/mg with ratios of the YW/USP titers of at least 6, capable of being above about 65,

c) a mucopolysaccharidic composition formed by a mixture of chains having an average molecular weight of about 3000 to 6000 daltons and comprising end units having a 2,5-anhydromanno structure, preferably 2,5-anhydromannitol or 2,5-anhydromannonic acid groups;

d) a mucopolysaccharidic composition of the type of those obtained by autoregulated depolymerization of heparin, advantageously formed by a mixture of chains 98% of which have a 2,5-anhydrymanno structure;

e) an oligosaccharide having no more than 8 saccharidic moieties, having a specific affinity of ATIII and a ratio of the YW/USP titers of at least 30, more particularly an octasaccharide of structure ABCDEFGH

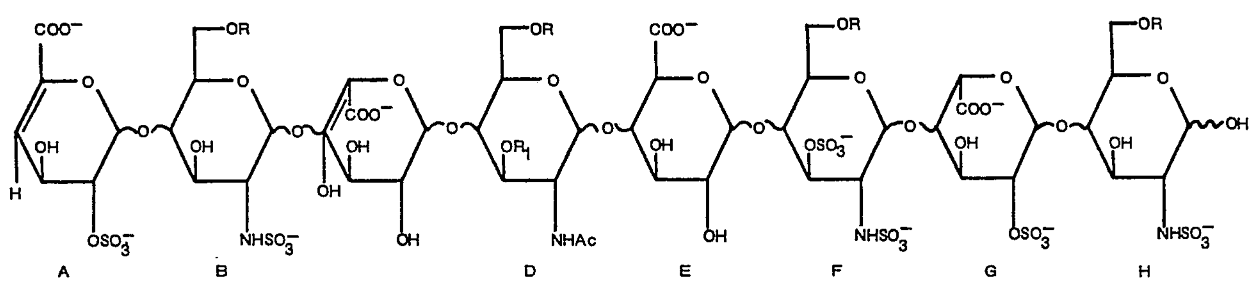

the USP titer of said octasaccharide being practically null and the Yin Wessler titer thereof ten times higher than the one of heparin, even capable of reaching values of the order of 2000 u/mg;

f) an hexasaccharidic composition of high homogeneity based on chains of structure C'EDFGH

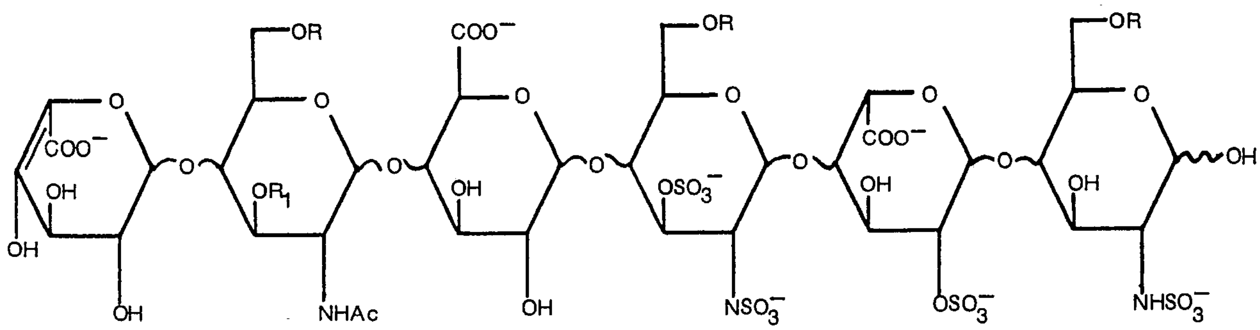

C' D E F G H

said composition having a Yin-Wessler titer higher than 2000 u/mg and a USP or APTT titer of the order of 10 or lower;

g) an oligosaccharide having a low molecular weight such as those obtained by the synthetic route, particularly the pentasaccharide of structure DEFGH of formula

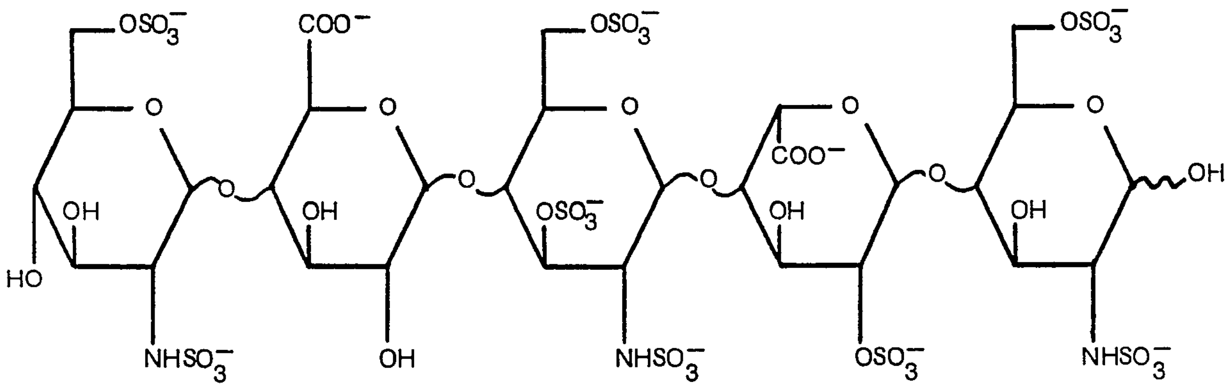

h) an oligosaccharide comprising 4 to 12 moieties, selected between amino sugar — uronic acid moieties or the reverse, and containing a tetrasaccharidic enchainment of structure DEFG

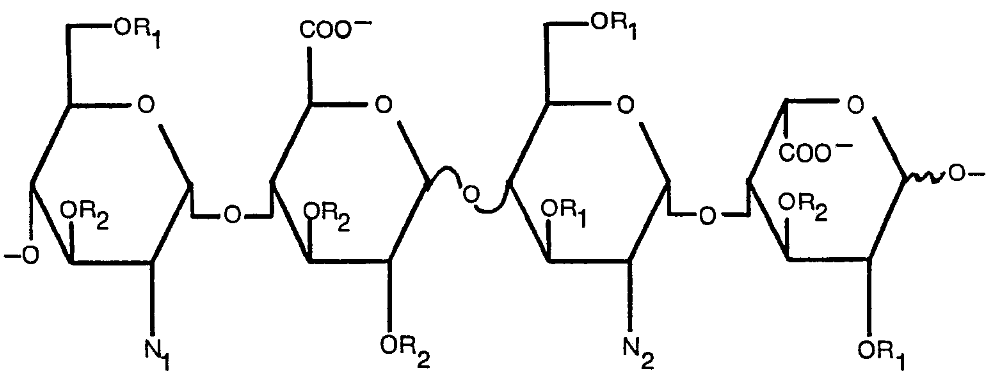

in which:

the radicals $R_1$, identical or different from one another, represent an inorganic anion, in particular a sulfate group or a phosphate group,

$R_2$ has one of the meaning given for $R_1$ or represent an hydrogen atom,

$N_1$ and $N_2$, identical or different from one another, represent a fonctional amine group, in particular in the form of a salt with an inorganic anion such as defined above, or substituted by an acyl group — $COR_3$ where $R_3$ represents an alkyl radical and their salts;

i) an oligosaccharide or a fraction such as above defined in form of a conjugate with ATIII or in form of affinity complexes.

3. Use according to claim 2, wherein the oligosaccharides correspond to a mucopolysaccharidic fraction according to claim 2a having in deuterated solution, in NMR (13C)

23.4 ppm: $CH_3$ (NH-acetyl group),

54.7 ppm: carbon at the 2 position of glucosamine acetyl units,

59.2 and 58.8 ppm: carbon at the 2 position of glucosamine sulphate units (the peak in the region of 58 ppm does not appear under identical measuring conditions with heparin),

61.7 ppm: carbon at the 6 position with —OH groups,

67.4 ppm: carbon at the 6 position with —O-sulfate groups,

98.3** ppm: carbon at the 1 position,

100.00 ppm**: carbon at the 1 position of sulfated iduronic acid units,

103 ppm: carbon at the 1 position of the glucuronic acid units.

4. Use according to any one of claims 1 to 3, wherein said oligosaccharide is a mucopolysaccharidic fraction having an average molecular weight of the order of 4500 and a ratio of the YW/USP titers close to 3.55.

5. Use according to any one of the preceding claims, wherein said oligosaccharide is used in association with an appropriate carrier for the oral administration.

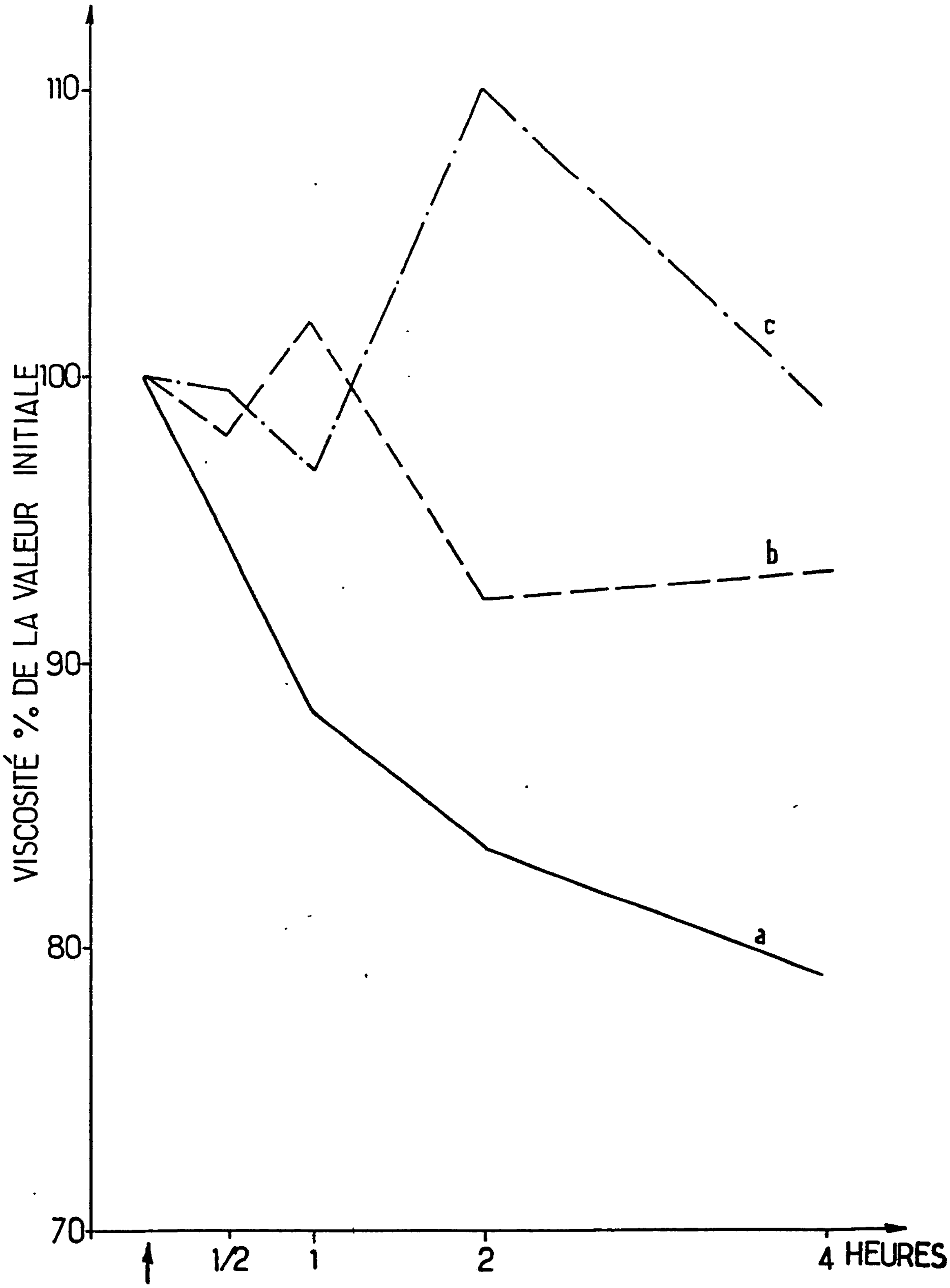
VISCOSITÉ % DE LA VALEUR INITIALE
110
100
90
80
70
c
b
a
1/2
1
2
4 HEURES